# EUROPEAN PATENT APPLICATION

(11) **EP 2 952 133 A1**
(43) Date of publication of application: **09.12.2015**
(21) Application number: 14880383.6
(22) Date of filing: 16.10.2014
(51) Int. Cl.: A61B 1/12

(54) **CLEANING AND STERILIZING METHOD USING ENDOSCOPE CLEANING AND STERILIZING DEVICE**

(30) Priority: 07.03.2014 JP 2014045319
(71) Applicant: OLYMPUS CORPORATION, Shibuya-ku Tokyo 151-0072 (JP)
(72) Inventor: AKAHORI, Hiromasa, Tokyo 151-0072 (JP)
(74) Representative: Schicker, Silvia
(86) International application number: PCT/JP2014/077491
(87) International publication number: WO 2015/132998

(57) **Abstract**

A cleaning/disinfecting method includes a step 10 of filling a first liquid storing section with a first liquid, a step 11 of supplying, by a set amount, a second liquid to a second liquid storing section, a step 12 of moving the first liquid and the second liquid back and forth or circulating the first liquid and the second liquid between the first liquid storing section and the second liquid storing section to thereby create mixed liquid of the first liquid and the second liquid, a step 13 of detecting a ratio of the first liquid or the second liquid included in the mixed liquid, and a step 14 of comparing the ratio with a specified value to thereby check a contact degree of the mixed liquid with the first liquid storing section.

## Description

### Technical Field

The present invention relates to a cleaning/disinfecting method using an endoscope cleaning/disinfecting apparatus that cleans and disinfects an endoscope.

### Background Art

In an endoscope cleaned and disinfected by an endoscope cleaning/disinfecting apparatus, not only an outer surface but also insides of known endoscope conduits such as a suction conduit, a front water feeding conduit, and an air/water feeding conduit provided in the endoscope are cleaned and disinfected.

Note that cleaning liquid and disinfecting liquid used in cleaning and disinfection exhibit effects by coming into contact with stains.

Therefore, in order to surely clean and disinfect the insides of the endoscope conduits, it is necessary to surely bring the cleaning liquid and the disinfecting liquid into contact with conduit inner walls. As the cleaning liquid and the disinfecting liquid, cleaning liquid and disinfecting liquid diluted to a predetermined concentration by water is used.

Japanese Patent Application Laid-Open Publication No. 8-197027 discloses a configuration and a method for measuring parameters such as a pressure change, a change in a reflection amount or an absorption amount of light, and a light path change that occur when liquid comes into contact with an inner wall of a liquid storing section, comparing a result of the measurement and data during normal time when the liquid is surely in contact with the inner wall of the liquid storing section, and confirming that the liquid is surely in contact with the inner wall of the liquid storing section.

With the method disclosed in Japanese Patent Application Laid-Open Publication No. 8-197027, it can only be detected that cleaning liquid enters a container in which an object to be cleaned is stored. For example, it is difficult to detect whether the cleaning liquid comes into contact with an entire target region of the object to be cleaned in the container.

The present invention has been devised in view of the above problem and it is an object of the present invention to provide a cleaning/disinfecting method using an endoscope cleaning/disinfecting apparatus that can easily confirm that cleaning liquid and disinfecting liquid come into contact with an inner wall of a liquid storing section.

### Disclosure of Invention

### Means for Solving the Problem

A cleaning/disinfecting method using an endoscope cleaning/disinfecting apparatus in an aspect of the present invention includes: a step of filling a first liquid storing section disposed in the endoscope cleaning/disinfecting apparatus with a first liquid; a step of supplying, by a set amount, a second liquid to a second liquid storing section disposed in the endoscope cleaning/disinfecting apparatus and communicating with the first liquid storing section; a step of moving the first liquid and the second liquid back and forth or circulating the first liquid and the second liquid between the first liquid storing section and the second liquid storing section to thereby create mixed liquid of the first liquid and the second liquid; a step of detecting a ratio of the first liquid or the second liquid included in the mixed liquid; and a step of comparing the ratio with a specified value to thereby check a contact degree of the mixed liquid with the first liquid storing section.

### Brief Description of the Drawings

Fig. 1 is a diagram schematically showing a configuration of an endoscope cleaning/disinfecting apparatus used in a cleaning/disinfecting method of a first embodiment;
Fig. 2 is a flowchart showing a cleaning/disinfecting process using the endoscope cleaning/disinfecting apparatus shown in Fig. 1;
Fig. 3 is a flowchart showing the cleaning/disinfecting method using the endoscope cleaning/disinfecting apparatus of the first embodiment;
Fig. 4 is a flowchart showing a specific example of the cleaning/disinfecting method using the endoscope cleaning/disinfecting apparatus shown in Fig. 3;
Fig. 5 is a diagram schematically showing an endoscope including, on an inside, an endoscope conduit serving as a modification of a first liquid storing section shown in Fig. 1;
Fig. 6 is a diagram schematically showing a configuration of an endoscope cleaning/disinfecting apparatus used in a cleaning/disinfecting method of a second embodiment;
Fig. 7 is a diagram schematically showing a configuration in which an inside of a sealed container is filled with water when a container including a filter shown in Fig. 6 is the sealed container;
Fig. 8 is a diagram schematically showing a configuration in which an unsealed container is filled with water when the container including the filter shown in Fig. 6 is the unsealed container;
Fig. 9 is a flowchart showing a specific example of the cleaning/disinfecting method using the endoscope cleaning/disinfecting apparatus of the second embodiment; and
Fig. 10 is a diagram showing an example of an internal configuration of the endoscope cleaning/disinfecting apparatus.

### Best Mode for Carrying Out the Invention

Embodiments of the present invention are explained below with reference to the drawings. It should be noted that the drawings are schematic and relations between thicknesses and widths of respective members, ratios of thicknesses of the respective members, and the like are sometimes different from real ones. It goes without saying that portions having different relations and different ratios of dimensions are included among the drawings.

### (First Embodiment)

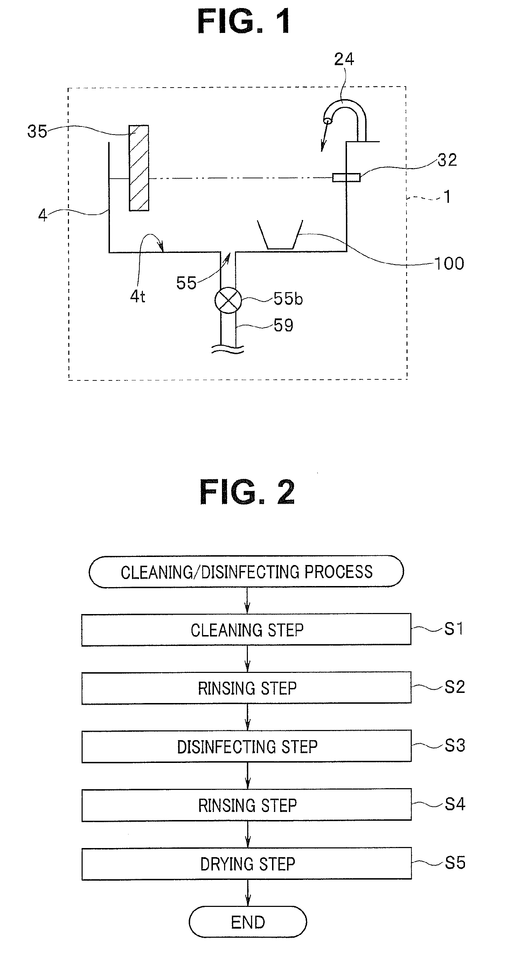

Fig. 1 is a diagram schematically showing a configuration of an endoscope cleaning/disinfecting apparatus used in a cleaning/disinfecting method of the present embodiment.

First, prior to explanation of the cleaning/disinfecting method using the endoscope cleaning/disinfecting apparatus of the present embodiment, a configuration of the endoscope cleaning/disinfecting apparatus of the present embodiment is briefly explained.

As shown in Fig. 1, an endoscope cleaning/disinfecting apparatus 1 includes a second liquid storing section 4 in which a first liquid storing section 100 is housed and that communicates with the first liquid storing section 100.

Note that, in the embodiment explained below, a cleaning/disinfecting tank is explained as an example of the second liquid storing section 4. Therefore, for convenience of explanation, the cleaning/disinfecting tank is also denoted by reference numeral 4.

The first liquid storing section 100 is cleaned and disinfected in the cleaning/disinfecting tank 4. In the present embodiment, a concave member, that is, a glass 100 is explained as an example of the first liquid storing section. In the cleaning/disinfecting tank 4, a discharge port 55 for discharging liquid from the cleaning/disinfecting tank 4 is provided in a bottom surface 4t. A liquid discharge conduit 59, in which a valve 55b is provided in a halfway position, is connected to the discharge port 55.

In the cleaning/disinfecting tank 4, a water level sensor 32 that detects a water level of liquid supplied to the cleaning/disinfecting tank 4 and a concentration sensor 35 that measures concentration of the liquid supplied to the cleaning/disinfecting tank 4 are provided.

Further, in the endoscope cleaning/disinfecting apparatus 1, a nozzle 24 that supplies a first liquid and a second liquid to the cleaning/disinfecting tank 4 is provided.

Note that, in the following explanation, in the present embodiment, disinfecting liquid is explained as an example of the first liquid. Water is explained as an example of the second liquid.

Next, a cleaning/disinfecting process using the endoscope cleaning/disinfecting apparatus shown in Fig. 1 is briefly explained with reference to Fig. 2. Fig. 2 is a flowchart showing the cleaning/disinfecting process using the endoscope cleaning/disinfecting apparatus shown in Fig. 1.

First, when the glass 100 is cleaned and disinfected in the cleaning/disinfecting tank 4, in step S1, cleaning liquid is supplied from the nozzle 24 to the cleaning/disinfecting tank 4 and a cleaning/disinfecting step for the glass 100 is performed.

Subsequently, after the cleaning liquid is discharged from the cleaning/disinfecting tank 4 via the discharge port 55, in step S2, water is supplied from the nozzle 24 to the cleaning/disinfecting tank 4 and a rinsing step for the glass 100 is performed.

Thereafter, after the water is discharged from the cleaning/disinfecting tank 4 via the discharge port 55, in step S3, disinfecting liquid is supplied from the nozzle 24 to the cleaning/disinfecting tank 4 and a disinfecting step for the glass 100 is performed. Note that, in this disinfecting step, a water solution of peracetic acid, glutaraldehyde, or the like is used as the disinfecting liquid.

Subsequently, after the disinfecting liquid is discharged from the cleaning/disinfecting tank 4 via the discharge port 55, in step S4, water is supplied from the nozzle 24 to the cleaning/disinfecting tank 4 and a rinsing step for the glass 100 is performed.

Finally, after the water is discharged from the cleaning/disinfecting tank 4 via the discharge port 55, in step S5, alcohol or gas for water removal is supplied from the nozzle 24 to the cleaning/disinfecting tank 4 and a drying step is performed.

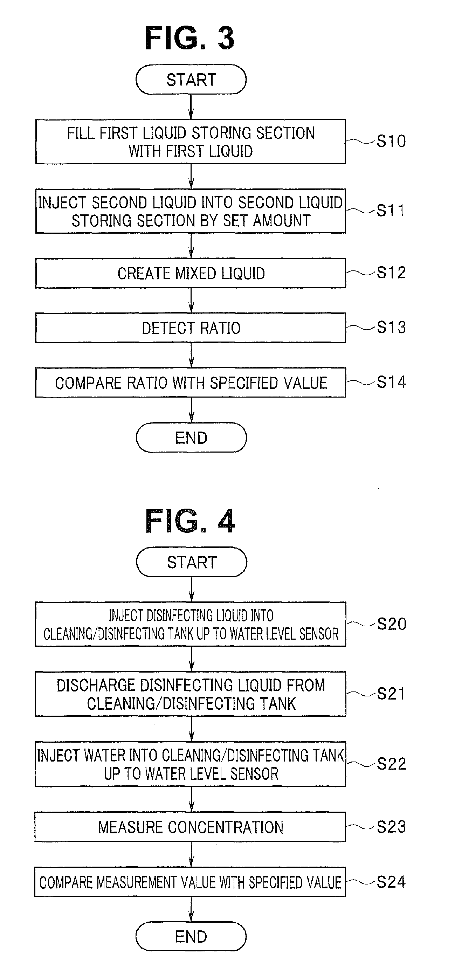

Next, a cleaning/disinfecting method using the endoscope cleaning/disinfecting apparatus of the present embodiment performed in the disinfecting step of step S3 in Fig. 2, more specifically, a method of confirming that disinfecting liquid diluted to predetermined concentration comes into contact with an inner wall of the glass 100 is roughly explained with reference to Fig. 3.

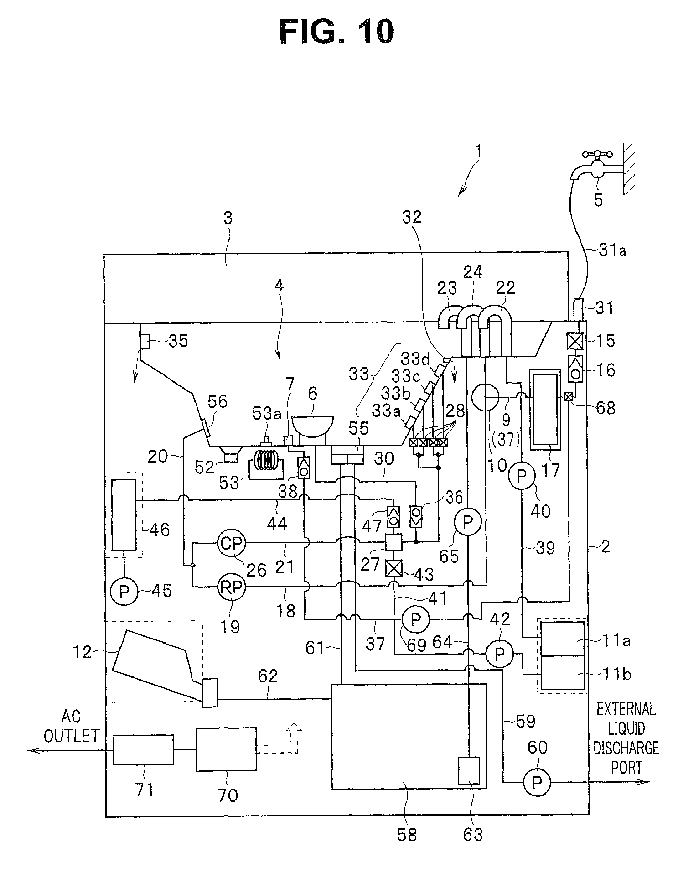

Fig. 3 is a flowchart showing the cleaning/disinfecting method using the endoscope cleaning/disinfecting apparatus of the present embodiment. Note that the flowchart of Fig. 3 explained below is performed by a control section 70 (see Fig. 10) explained below included in the endoscope cleaning/disinfecting apparatus 1.

As shown in Fig. 3, first, in step S10, the control section 70 fills the glass 100 with disinfecting liquid. Subsequently, in step S11, the control section 70 injects water into the cleaning/disinfecting tank 4 by a set amount.

Thereafter, in step S 12, the control section 70 moves the disinfecting liquid and the water back and forth or circulates the disinfecting liquid and the water between the glass 100 and the cleaning/disinfecting tank 4 to thereby create diluted disinfecting liquid, that is, mixed liquid of the disinfecting liquid and the water.

Subsequently, in step S 13, the control section 70 detects a ratio of the disinfecting liquid included in the mixed liquid. Finally, in step S 14, the control section 70 compares the ratio of the disinfecting liquid with a specified value to thereby check a contact degree of the disinfecting liquid with the glass 100.

Next, a more specific example of the cleaning/disinfecting method using the endoscope cleaning/disinfecting apparatus shown in Fig. 3 is explained with reference to Fig. 4.

Fig. 4 is a flowchart showing the specific example of the cleaning/disinfecting method using the endoscope cleaning/disinfecting apparatus shown in Fig. 3. Note that the flowchart of Fig. 4 explained below is also performed by the control section 70 (see Fig. 10) explained below included in the endoscope cleaning/disinfecting apparatus 1.

As shown in Fig. 4, first, during the disinfecting step of step S3 shown in Fig. 2, in step S20, the control section 70 injects disinfecting liquid from the nozzle 24 into the cleaning/disinfecting tank 4 up to the water level sensor 32. The water level sensor is set in a position higher than an opening position of the glass 100. Consequently, step S10 in Fig. 3 for filling the glass 100 with the disinfecting liquid is performed.

Subsequently, in step S21, the control section 70 discharges the disinfecting liquid from the cleaning/disinfecting tank 4 via the discharge port 55. Note that, when the disinfecting liquid is discharged, the glass 100 is kept filled with the disinfecting liquid.

Thereafter, during the rinsing step of step S4 shown in Fig. 2 or in a separate step, in step S22, the control section 70 injects water from the nozzle 24 into the cleaning/disinfecting tank 4 up to the water level sensor 32. Consequently, step S11 in Fig. 3 is performed.

After the injection of the water, in the cleaning/disinfecting tank 4, the water mixes with the disinfecting liquid filled in the glass 100. As a result, the disinfecting liquid is diluted and concentration of the disinfecting liquid decreases. Consequently, the creation of the mixed liquid in step S12 in Fig. 3 is performed.

Thereafter, in step S23, the concentration sensor 35 is used and concentration of the mixed liquid is measured. When the concentration of the mixed liquid is measured, a ratio of the disinfecting liquid included in the mixed liquid in step S 13 in Fig. 3 is detected. Note that, as parameters to be detected, a pH value, electric conductivity, and the like are also included besides density of a disinfecting liquid component.

Finally, in step S24, the step S 14 in Fig. 3 for comparing a measured value in step S23 and a specified value of concentration in the case in which the disinfecting liquid is filled in the glass 100 and the water is injected in the cleaning/disinfecting tank 4 up to the water level sensor 32 is performed.

As a result of the comparison, on one hand, if the measured value is equal to or larger than the specified value, it can be determined that the disinfecting liquid is filled in the glass 100.

That is, it can be determined that the disinfecting liquid is surely in contact with the inner wall of the glass 100. It can also be determined that the mixed liquid is also surely in contact with the inner wall of the glass 100.

On the other hand, if the measured value is lower than the specified value, it can be determined that a sufficient amount of the disinfecting liquid is not injected into the glass 100 in step S20 or a place where the disinfecting liquid is not in contact with the inner wall of the glass 100 is present because of air bubbles or the like.

That is, it can be determined that the disinfecting liquid is not sufficiently in contact with the inner wall of the glass 100. Further, it can be determined that the mixed liquid is not sufficiently in contact with the inner wall of the glass 100 either. That is, it can be determined that disinfection is defective.

Note that it can be determined whether the disinfecting liquid is in contact with the inner wall of the glass 100 by such a method using the concentration measurement because capacities of the glass 100 and the cleaning/disinfecting tank 4 are known beforehand and are fixed.

As explained above, in the present embodiment, the disinfecting liquid is filled in the glass 100 housed in the cleaning/disinfecting tank 4, thereafter, the water is injected into the cleaning/disinfecting tank 4 up to the water level sensor 32 to create the mixed liquid, concentration of the mixed liquid is measured using the concentration sensor 35, and, if the measured value is lower than the specified value, it is determined that the disinfecting liquid is not sufficiently in contact with the inner wall of the glass 100.

Therefore, using an existing configuration of the endoscope cleaning/disinfecting apparatus 1, it is possible to confirm that the mixed liquid is surely in contact with the inner wall of the glass 100 during the cleaning/disinfecting process shown in Fig. 2 with a simple method of only measuring concentration of the mixed liquid in the cleaning/disinfecting tank 4 using the concentration sensor 35 and comparing the concentration with the specified value.

Consequently, it is possible to provide the cleaning/disinfecting method using the endoscope cleaning/disinfecting apparatus that can surely confirm in each cleaning/disinfecting process that the mixed liquid comes into contact with the inner wall of the liquid storing section.

Note that a modification is explained below.

In the present embodiment explained above, the disinfecting liquid is explained as the example of the first liquid. However, not only this, but it goes without saying that the first liquid may be cleaning liquid.

The disinfecting liquid is explained as the example of the first liquid and the water is explained as the example of the second liquid. However, not only this, but the first liquid may be the water and the second liquid may be the disinfecting liquid.

In this case, in the rinsing step of step S2 in Fig. 2, the water may be filled in the glass 100. Thereafter, in the disinfecting step of step S3, the disinfecting liquid may be injected into the cleaning/disinfecting tank 4 up to the water level sensor 32 to create the mixed liquid and concentration of the mixed liquid may be measured and compared with the specified value.

When the concentration of the mixed liquid is compared with the specified value, if a measured value of the concentration of the mixed liquid is higher than the specified value, it can be determined that the water is not in contact with the inner wall of the glass 100. Therefore, it can be determined that the mixed liquid is not sufficiently in contact with the inner wall of the glass 100 either. It can be determined that disinfection is defective.

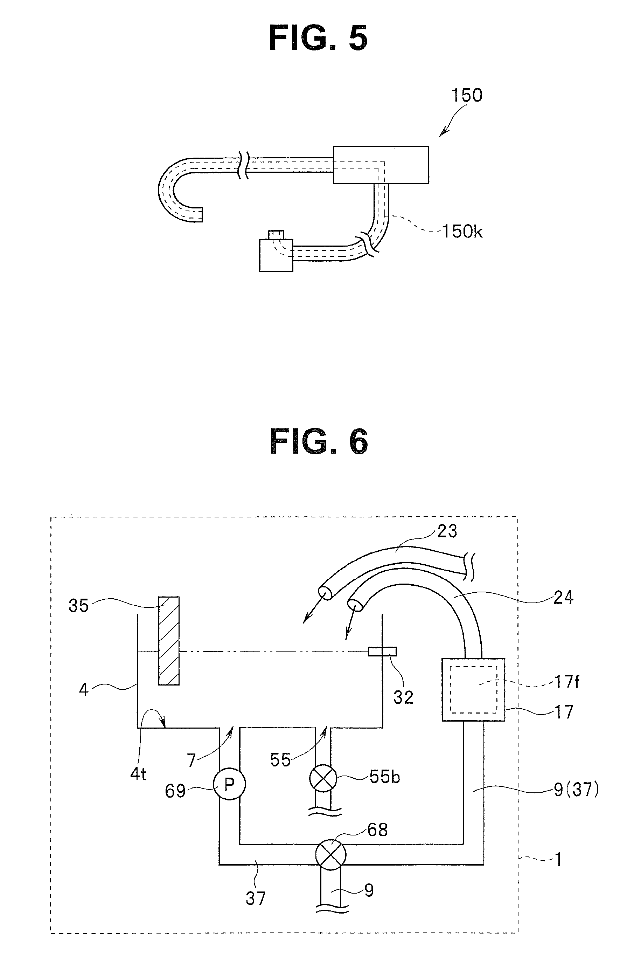

Another modification is explained with reference to Fig. 5. Fig. 5 is a diagram schematically showing an endoscope including, on an inside, an endoscope conduit serving as a modification of the first liquid storing section shown in Fig. 1.

In the embodiment explained above, the glass 100 is explained as the example of the first liquid storing section.

Not only this, but, as shown in Fig. 5, the first liquid storing section may be an endoscope conduit 150k in an endoscope 150 housed in the cleaning/disinfecting tank 4.

Note that, in Fig. 5, in order to simplify the drawing, only one endoscope conduit 150k is shown. However, actually, a plurality of the endoscope conduits 150k are provided.

That is, if the glass 100 of the present embodiment explained above is replaced with the endoscope conduit 150k, as in the present embodiment explained above, it is possible to surely confirm that the mixed liquid is in contact with an inner wall of the endoscope conduit 150k.

Note that a capacity of the endoscope conduit 150k is different depending on a model or the like. Therefore, when an operator checks contact of the mixed liquid with the inner wall of the endoscope conduit 150k, the operator desirably inputs the capacity to the endoscope cleaning/disinfecting apparatus 1 beforehand.

### (Second Embodiment)

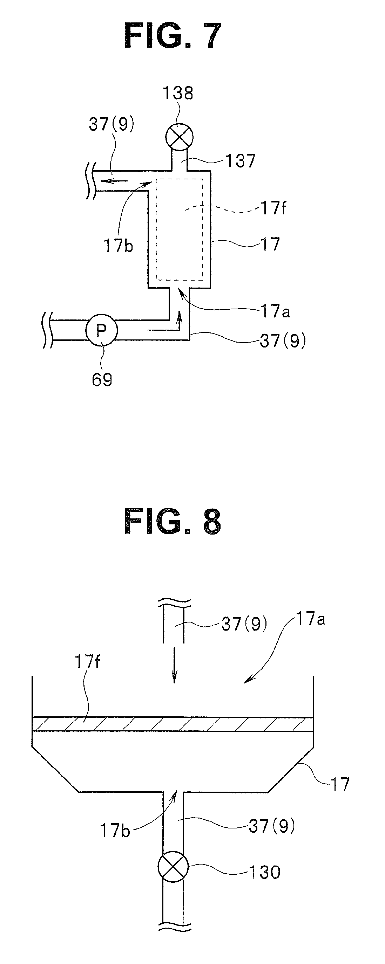

Fig. 6 is a diagram schematically showing a configuration of an endoscope cleaning/disinfecting apparatus used in a cleaning/disinfecting method of the present embodiment. Fig. 7 is a diagram schematically showing a configuration in which an inside of a sealed container is filled with water when a container including a filter shown in Fig. 6 is the sealed container. Fig. 8 is a diagram schematically showing a configuration in which an unsealed container is filled with water when the container including the filter shown in Fig. 6 is the unsealed container.

A cleaning method using the endoscope cleaning/disinfecting apparatus of the second embodiment is different from the cleaning method using the endoscope cleaning/disinfecting apparatus of the first embodiment explained above in that the first liquid storing section is a container including a filter that filters water supplied to the cleaning/disinfecting tank.

Therefore, only this difference is explained. Components same as the components in the first embodiment are denoted by the same reference numerals and signs and explanation of the components is omitted.

First, prior to explanation of the cleaning/disinfecting method using the endoscope cleaning/disinfecting apparatus of the present embodiment, only portions of a configuration of the endoscope cleaning/disinfecting apparatus of the present embodiment different from those of the first embodiment are briefly explained.

As shown in Fig. 6, besides the discharge port 55, a circulation port 7 is provided in the bottom surface 4t of the cleaning/disinfecting tank 4. A filter circulation conduit 37 communicating with the nozzle 24 is connected to the circulation port 7.

Note that, in halfway positions of the filter circulation conduit 37, a pump 69, a valve 68, and a first liquid storing section 17 for circulation are provided. Note that the first liquid storing section 17 communicates with the cleaning/disinfecting tank 4 via the filter circulation conduit 37.

A water supply conduit 9 communicating with a water tap or the like is connected to the valve 68. Note that a downstream side of the filter circulation conduit 37, that is, a nozzle 24 side also functions as the water supply conduit 9.

Therefore, on one hand, when the circulation port 7 and the nozzle 24 communicate with each other via the valve 68, liquid in the cleaning/disinfecting tank 4 is supplied from the nozzle 24 to the cleaning/disinfecting tank 4 again via the filter circulation conduit 37 by driving of the pump 69. That is, the liquid in the cleaning/disinfecting tank 4 is circulated.

On the other hand, when the water supply conduit 9 and the nozzle 24 communicate with each other via the valve 68, water from the water tap is supplied from the nozzle 24 to the cleaning/disinfecting tank 4.

Note that, in the present embodiment, a container including a filter 17f that filters tap water from the water tap and removes foreign matters, bacteria, and the like is explained as an example of the first liquid storing section 17. Therefore, for convenience of explanation, the container is also denoted by reference numeral 17.

The container 17 has a configuration that can be surely filled by the first liquid or the second liquid, that is, a capacity of the container 17 can be replaced with the first liquid or the second liquid and can be always filled by the first liquid or the second liquid during liquid supply.

More specifically, as shown in Fig. 7, when the container 17 is a sealed container, in the container 17, an introducing port 17a, an outlet 17b, and an air outlet 137 including a switching valve 138 are provided.

Note that, when the outlet 17b is provided at a top in a gravity direction, the outlet 17b can also be used as the outlet 137. The switching valve 138 is configured to be opened only in air bleeding. Further, the outlet 137 is provided at the top in the gravity direction.

Therefore, the first liquid or the second liquid supplied from an upstream side of the filter circulation conduit 37 or from the water supply conduit 9 is introduced into the container 17 via the introducing port 17a and, after passing through the filter 17f, flows from the outlet 17b to a downstream side of the filter circulation conduit 37.

Since the container 17 includes such a configuration, when the first liquid or the second liquid is supplied to the container 17, an inside of the container 17 can be filled with the first liquid or the second liquid.

As shown in Fig. 8, when the container 17 is an unsealed container, the container 17 includes the introducing port 17a at an upper part in the gravity direction, includes, in a lower part in the gravity direction, the outlet 17b communicating with the downstream side of the filter circulation conduit 37, and has a funnel shape opened in the introducing port 17a.

Note that the filter circulation conduit 37 includes a switching valve 130 further on the downstream side than the outlet 17b.

The container 17 includes the filter 17f on an inside. Therefore, the first liquid or the second liquid supplied from the upstream side of the filter circulation conduit 37 or from the water supply conduit 9 is introduced into the container 17 via the introducing port 17a and, after passing through the filter 17f, when the switching valve 130 is open, flows from the outlet 17b to the downstream side of the filter circulation conduit 37.

Note that, since the introducing port 17a is opened, air does not accumulate in the container 17. That is, the container 17 does not have a structure from which the air does not escape like an eaves shape.

Further, a water level sensor may be provided in the container 17 such that the first liquid or the second liquid can be filled in the container 17. The container 17 may have an overflow structure.

Further, the supply of the first liquid or the second liquid from the upstream side of the filter circulation conduit 37 or from the water supply conduit 9 into the container 17 via the introducing port 17a is performed by, for example, reducing a liquid feed amount to prevent a water surface from rippling, supplying the first liquid or the second liquid from a position apart from the water level sensor or an overflow position, or supplying the first liquid or the second liquid from the outlet 17b side, although not shown in the figure.

Note that, when the water level sensor is used, the liquid feeding method for preventing the water surface from foaming is selected or low-foaming liquid is used as the first liquid or the second liquid.

Since the container 17 has such a configuration, when the first liquid or the second liquid is supplied to the container 17 by the pump 69, a tap water pressure, gravity, or the like, it is possible to fill the inside of the container 17 with the first liquid or the second liquid.

Further, in the endoscope cleaning/disinfecting apparatus 1, besides the nozzle 24 that supplies any one of the first liquid, the second liquid, and the mixed liquid of the first liquid and the second liquid to the cleaning/disinfecting tank 4, a nozzle 23 that supplies the first liquid or the second liquid is provided.

Note that, in the following explanation, in the present embodiment, water is explained as an example of the first liquid and disinfecting liquid is explained as an example of the second liquid.

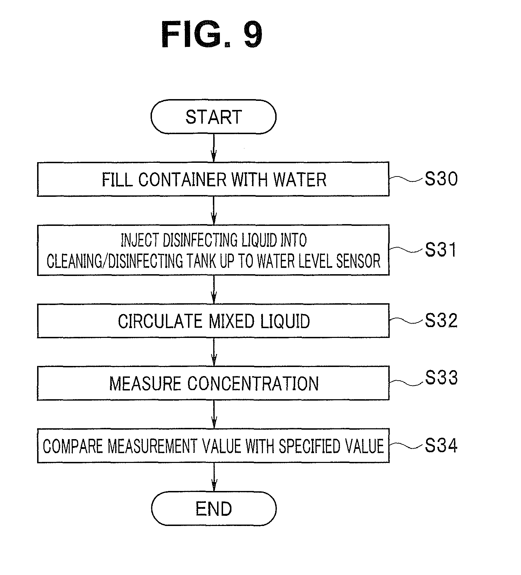

Next, a more specific example of the cleaning/disinfecting method using the endoscope cleaning/disinfecting apparatus of the present embodiment is explained with reference to Fig. 9. Fig. 9 is a flowchart showing the specific example of the cleaning/disinfecting method using the endoscope cleaning/disinfecting apparatus of the present embodiment. Note that the flowchart of Fig. 9 explained below is also performed by the control section 70 (see Fig. 10) explained below included in the endoscope cleaning/disinfecting apparatus 1.

As shown in Fig. 9, first, during the rinsing step of step S2 shown in Fig. 2, in step S30, the control section 70 fills the container 17 with water. Consequently, step S10 in Fig. 3 for filling the container 17 with the water is performed.

Subsequently, in step S31, the control section 70 injects disinfecting liquid from the nozzle 23 into the cleaning/disinfecting tank 4 up to the water level sensor 32. Consequently, step S11 in Fig. 3 is performed.

Thereafter, in step S32, the control section 70 drives the pump 69 to thereby circulate the disinfecting liquid to the cleaning/disinfecting tank 4. When the disinfecting liquid is circulated, the water and the disinfecting liquid in the container 17 are mixed when the disinfecting liquid passes through the container 17.

As a result, the disinfecting liquid is diluted and concentration of the disinfecting liquid decreases. Consequently, the creation of mixed liquid in step S12 in Fig. 3 is performed. The mixed liquid is circulated to the cleaning/disinfecting tank 4.

Thereafter, in step S33, the concentration sensor 35 is used and concentration of the mixed liquid is measured. When the concentration of the mixed liquid is measured, a ratio of the disinfecting liquid included in the mixed liquid in step S 13 in Fig. 3 is detected. Note that a pH value, electric conductivity, and the like are also included in the concentration of the mixed liquid besides density of a disinfecting liquid component.

Finally, in step S34, the step S14 in Fig. 3 for comparing a measured value in step S33 and a specified value of concentration in the case in which the water is filled in the container 17 and the disinfecting liquid is filled in the cleaning/disinfecting tank 4 up to the water level sensor 32 is performed.

As a result of the comparison, on one hand, if the measured value is equal to or smaller than the specified value, it can be determined that the water is filled in the container 17.

That is, it can be determined that the water is surely in contact with the inner wall of the container 17. It can be determined that the mixed liquid is also surely in contact with the inner wall of the container 17.

On the other hand, if the measured value is higher than the specified value, it can be determined that a sufficient amount of the water is not injected into the container 17 in step S30 or a place where the water is not in contact with the inner wall of the container 17 is present because of air bubbles or the like.

That is, it can be determined that the water is not in contact with the inner wall of the container 17. Further, it can be determined that the mixed liquid is not sufficiently in contact with the inner wall of the container 17 either. That is, it can be determined that disinfection is defective.

Note that it can be determined whether the water or the mixed liquid is in contact with the inner wall of the container 17 by such a method using the concentration measurement because capacities of the container 17 and the cleaning/disinfecting tank 4 are known beforehand and are fixed.

As explained above, in the present embodiment, the water is filled in the container 17 that communicates with the cleaning/disinfecting tank 4 via the filter circulation conduit 37, thereafter, the disinfecting liquid is injected into the cleaning/disinfecting tank 4 up to the water level sensor 32 to create the mixed liquid, concentration of the mixed liquid is measured using the concentration sensor 35, and, if the measured value is higher than the specified value, it is determined that the water and the mixed liquid is not sufficiently in contact with the inner wall of the container 17.

Therefore, using an existing configuration of the endoscope cleaning/disinfecting apparatus 1, it is possible to confirm that the mixed liquid is surely in contact with the inner wall of the container 17 during the cleaning/disinfecting process shown in Fig. 2 with a simple method of only measuring concentration of the mixed liquid in the cleaning/disinfecting tank 4 using the concentration sensor 35 and comparing the concentration with the specified value.

Consequently, it is possible to provide the cleaning/disinfecting method using the endoscope cleaning/disinfecting apparatus that can surely confirm in each cleaning/disinfecting process that the mixed liquid comes into contact with the inner wall of the liquid storing section.

The specified value in the present invention may be inputted in advance in the endoscope cleaning/disinfecting apparatus or may be a value inputted from the outside or a value calculated in the endoscope cleaning/disinfecting apparatus on the basis of information inputted from the outside.

As means for inputting a value from the outside, for example, the endoscope cleaning/disinfecting apparatus may read a specified value or information stored in an information source incidental to an endoscope such as an RF-ID or a barcode of the endoscope to input the specified value or the information to the endoscope cleaning/disinfecting apparatus. In this case, it is desirable that an RF-ID reader or a barcode reader is provided in or connectable to the endoscope cleaning/disinfecting apparatus.

As another means, the specified value or the like may be able to be inputted by a human hand. In this case, it is desirable that an input interface is provided in or connectable to the endoscope cleaning/disinfecting apparatus.

As another means, the endoscope cleaning/disinfecting apparatus may be able to download a latest specified value list from accessible information source such as cloud. In this case, it is desirable that a communication function is provided in or connectable to the endoscope cleaning/disinfecting apparatus.

Note that a modification is explained below.

In the present embodiment explained above, the disinfecting liquid is explained as the example of the second liquid. However, not only this, but it goes without saying that the second liquid may be cleaning liquid.

In the present embodiment as well, the water is explained as the example of the first liquid and the disinfecting liquid is explained as the example of the second liquid. However, not only this, but the first liquid may be the disinfecting liquid and the second liquid may be the water.

In this case, in the disinfecting step of step S3 in Fig. 2, the disinfecting liquid may be filled in the container 17. Thereafter, in the rinsing step of step S4 or in a separate step, the water may be injected into the cleaning/disinfecting tank 4 up to the water level sensor 32 to create the mixed liquid and concentration of the mixed liquid may be measured and compared with the specified value.

When the concentration of the mixed liquid is compared with the specified value, if a measured value of the concentration of the mixed liquid is lower than the specified value, it can be determined that the water is not in contact with the inner wall of the container 17. Therefore, it can be determined that the mixed liquid is not sufficiently in contact with the inner wall of the container 17 either. It can be determined that disinfection is defective.

In the present embodiment explained above, the container 17 is explained as the example of the first liquid storing section. Not only this, but the first liquid storing section may be a conduit for circulating liquid to the cleaning/disinfecting tank 4 provided in the endoscope cleaning/disinfecting apparatus 1, for example, the filter circulation conduit 37.

That is, if the container 17 of the present embodiment explained above is replaced with the filter circulation conduit 37, as in the present embodiment explained above, it is possible to surely confirm that the mixed liquid is in contact with the inner wall of the filter circulation conduit 37.

Note that a conduit for circulating liquid to another cleaning/disinfecting tank 4 provided in the endoscope cleaning/disinfecting apparatus 1 may be a circulation conduit 20, a channel conduit 21, a liquid feeding conduit 18, and a case conduit 30 shown in Fig. 10.

An example of an internal configuration of the endoscope cleaning/disinfecting apparatus is briefly explained with reference to Fig. 10. Fig. 10 is a diagram showing the example of the internal configuration of the endoscope cleaning/disinfecting apparatus.

As shown in Fig. 10, a main part of the endoscope cleaning/disinfecting apparatus 1 is configured by an apparatus main body 2 and a top cover 3 connected to an upper part of the apparatus main body 2 via, for example, a not-shown hinge to be capable of opening and closing.

A water supply hose connecting port 31, to which a water tap 5 is connected via a tube 131a, communicates with one end of the water supply conduit 9.

The other end of the water supply conduit 9 is connected to a three-way electromagnetic valve 10. Halfway in the conduit, a water supply electromagnetic valve 15, a check valve 16, the valve 68, and the container 17 are interposed in order from the water supply hose connecting port 31 side.

The three-way electromagnetic valve 10 is connected to one end of the liquid feeding conduit 18 and switches, with a valve on the inside, communication of the water supply conduit 9 and the liquid feeding conduit 18 with the nozzle 24 exposed to the cleaning/disinfecting tank 4.

That is, the nozzle 24 communicates with one of the water supply conduit 9 and the liquid feeding conduit 18 according to a switching operation of the three-way electromagnetic valve 10. A liquid feeding pump 19 is interposed on the other end side of the liquid feeding conduit 18.

A circulation port 56 disposed in the cleaning/disinfecting tank 4 is connected to one end of the circulation conduit 20. The other end of the circulation conduit 20 branches into two to communicate with the other end of the liquid feeding conduit 18 and one end of the channel conduit 21.

The other end of the channel conduit 21 communicates with a port for air/water feeding/forceps 33 including respective ports 33a, 33b, 33c, and 33d.

The other ends of connection tubes, to which one end of an endoscope conduit included in an endoscope placed in the cleaning/disinfecting tank 4 is connected, are respectively connected to the respective ports 33a to 33d. Note that the first liquid storing section may be a connection tube.

In the channel conduit 21, halfway in the conduit, a channel pump 26, a channel block 27, and a channel electromagnetic valve 28 are respectively interposed in order from one end side. The other end of the case conduit 30, one end of which is connected to a cleaning case 6, is connected to the channel conduit 21 between the channel block 27 and the channel electromagnetic valve 28. A relief valve 36 is interposed in the case conduit 30.

A nozzle 22 exposed to the cleaning/disinfecting tank 4 is connected to one end of a cleaning agent conduit 39. The other end of the cleaning agent conduit 39 is connected to a detergent tank 11a.

In the cleaning agent conduit 39, halfway in the conduit, a detergent pump 40 for lifting a cleaning agent from the detergent tank 11a to the cleaning/disinfecting tank 4 is interposed.

An alcohol tank 11b is connected to one end of an alcohol conduit 41. The alcohol conduit 41 is connected to the channel block 27 to communicate with the channel conduit 21 in a predetermined manner.

In the alcohol conduit 41, an alcohol supply pump 42 for lifting alcohol from the alcohol tank 11b to the cleaning/disinfecting tank 4 and an electromagnetic valve 43 are interposed.

One end of an air conduit 44 for supplying air from an air pump 45, which can transfer gas, is connected to the channel block 27 to communicate with the channel conduit 21 in a predetermined manner.

The other end of the air conduit 44 is connected to the air pump 45. In halfway positions of the air conduit 44, a check valve 47 and an air filter 46, which is periodically replaced, are interposed.

In the discharge port 55 of the cleaning/disinfecting tank 4, the not-shown valve 55b explained above, which is capable of opening and closing, for discharging the cleaning liquid and the like to the outside and collecting the disinfecting liquid in a chemical tank 58 according to a switching operation is provided.

The discharge port 55 is connected to the other end of the liquid discharge conduit 59, one end of which is connected to and communicates with a not-shown water discharge hose connected to an external liquid discharge port. A water discharge pump 60 is interposed in the liquid discharge conduit 59.

The discharge port 55 is connected to one end of a chemical collection conduit 61. The other end of the chemical collection conduit 61 is connected to the chemical tank 58.

The chemical tank 58 is also connected to one end of a chemical supply conduit 62 such that a chemical, for example, disinfecting liquid is supplied from a chemical bottle 12 for the endoscope cleaning/disinfecting apparatus.

In the chemical tank 58, a one end portion of a chemical conduit 64, at the one end of which a suction filter 63 is provided, is housed in a predetermined manner.

The other end of chemical conduit 64 is connected to the nozzle 23 exposed to the cleaning/disinfecting tank 4. In a halfway position, a chemical pump 65 for lifting the disinfecting liquid from the chemical tank 58 to the cleaning/disinfecting tank 4 is interposed.

Note that, for example, two ultrasound transducers 52 and a heater 53 are disposed in a lower part of the bottom surface 4t of the cleaning/disinfecting tank 4. For temperature adjustment of the heater 53, a temperature sensor 53a is provided substantially in a center of the bottom surface 4t of the cleaning/disinfecting tank 4.

The circulation port 7 is provided in the bottom surface 4t. One end of the filter circulation conduit 37 is connected to the circulation port 7. The other end of the filter circulation conduit 37 is connected to the valve 68. Note that, in halfway positions of the filter circulation conduit 37, a check valve 38 and the pump 69 are interposed in order from the circulation port 7 side.

In the cleaning/disinfecting tank 4, as explained above, the water level sensor 32 and the concentration sensor 35 are provided.

On an inside of the endoscope cleaning/disinfecting apparatus 1, a power supply 71, to which electric power is supplied from an AC outlet on the outside, and the control section 70 electrically connected to the power supply 71 are provided. Various signals from a main operation panel and a sub operation panel, which are not shown in the figure, provided in the endoscope cleaning/disinfecting apparatus 1 are supplied to the control section 70. The control section 70 controls to drive the respective pumps, the respective electromagnetic valves, and the like explained above.

As explained above, the control section 70 performs measurement and comparison of concentration of mixed liquid using the water level sensor 32 and the concentration sensor 35.

This application is based upon and claims the benefit of priority from Japanese Patent Application No. 2014-045319 filed in Japan on March 7, 2014, the disclosed contents of which are incorporated in the specification, the claims, and the drawings.

## Claims

1. A cleaning/disinfecting method using an endoscope cleaning/disinfecting apparatus comprising:
a step of filling a first liquid storing section disposed in the endoscope cleaning/disinfecting apparatus with a first liquid;
a step of supplying, by a set amount, a second liquid to a second liquid storing section disposed in the endoscope cleaning/disinfecting apparatus and communicating with the first liquid storing section;
a step of moving the first liquid and the second liquid back and forth or circulating the first liquid and the second liquid between the first liquid storing section and the second liquid storing section to thereby create mixed liquid of the first liquid and the second liquid;
a step of detecting a ratio of the first liquid or the second liquid included in the mixed liquid; and
a step of comparing the ratio with a specified value to thereby check a contact degree of the mixed liquid with the first liquid storing section.

2. The cleaning/disinfecting method using the endoscope cleaning/disinfecting apparatus according to claim 1, wherein the first liquid is disinfecting liquid and the second liquid is water for dilution.

3. The cleaning/disinfecting method using the endoscope cleaning/disinfecting apparatus according to claim 1, wherein the first liquid is water for dilution and the second liquid is disinfecting liquid.

4. The cleaning/disinfecting method using the endoscope cleaning/disinfecting apparatus according to any one of claims 1 to 3, wherein the second liquid storing section is a cleaning/disinfecting tank provided in the cleaning/disinfecting apparatus.

5. The cleaning/disinfecting method using the endoscope cleaning/disinfecting apparatus according to claim 4, wherein the first liquid storing section is an endoscope conduit of an endoscope housed in the cleaning/disinfecting tank.

6. The cleaning/disinfecting method using the endoscope cleaning/disinfecting apparatus according to claim 4, wherein the first liquid storing section is a conduit of the cleaning/disinfecting apparatus for circulating liquid to the cleaning/disinfecting tank.

7. The cleaning/disinfecting method using the endoscope cleaning/disinfecting apparatus according to claim 4, wherein the first liquid storing section is a container including a filter that filters water supplied to the cleaning/disinfecting tank.
